# EUROPEAN PATENT SPECIFICATION

(11) **EP 2 211 685 B1**
(45) Date of publication and mention of the grant of the patent: **16.08.2017**
(21) Application number: 07821608.2
(22) Date of filing: 19.10.2007
(51) Int. Cl.: A61B 3/16

(54) **INTRAOCULAR PRESSURE MONITORING DEVICE**
VORRICHTUNG ZUR ÜBERWACHUNG DES AUGENINNENDRUCKS
DISPOSITIF DE SURVEILLANCE DE LA TENSION INTRAOCULAIRE

(43) Date of publication of application: 04.08.2010
(73) Proprietor: Sensimed SA, 1007 Lausanne (CH)
(72) Inventor: WISMER, Jean-Marc, CH-1006 Lausanne (CH)
(74) Representative: KATZAROV S.A.
(86) International application number: PCT/EP2007/061244
(87) International publication number: WO 2009/049686

(56) References cited:
- US-A1- 2004 186 366
- US-A1- 2005 268 726
- US-B1- 6 939 299

## Description

The present invention relates to a device for monitoring the intraocular pressure over a period of time. The present invention relates in particular to a device that can be placed on an eye to continuously monitor intraocular pressure over an extended period of time, for example 24 hours or more.

Glaucoma is a widespread disease characterized by an elevated intraocular pressure (IOP). This elevated IOP produces a gradual loss of peripheral vision. There is therefore a need to have a detailed knowledge of IOP in glaucoma patients in order to provide reliable diagnostics or for setting up new therapies.

Patent EP1401327 describes an intraocular pressure recording system comprising a soft contact lens and an active strain gage fixed to the contact lens. The active strain gage is placed at a distance from the center of the contact lens and is not in direct contact with the eye. The active strain gage comprises a portion having a circular arc shape which is situated around the center of the contact lens and thus allows measuring spherical deformations of the eyeball which are correlated with IOP.

The system of EP1401327 is not aggressive for the patient and doesn't necessitate to topically anesthetize the patient's eye and/or to surgically operate prior to testing. Furthermore, due to the fact that the strain gage is not in direct contact with the eye, the patient feels very comfortable and his vision remains almost completely unimpaired. In fact he has a similar feeling as a person wearing usual contact lenses.

The manufacturing of strain gages as the ones described in EP1401327, however, implies a relatively high quantity of waste material, for example in the case of Micro-Electro-Mechanical System (MEMS) gages manufactured in batches on a single substrate. Furthermore, manufacturing circular shaped elements in a reproducible manner is a difficult task, thus resulting in a relatively low rate of sufficiently reliable gages. The result of this is that reliable strain gages as described in EP1401327 are relatively expensive.

An aim of the invention is thus to provide a cheaper and still reliable intraocular pressure monitoring device.

This aim and other advantages are achieved by a device comprising the features of claim 1.

This aim is achieved in particular by an intraocular pressure monitoring device comprising a soft contact lens such as a silicone contact lens and an active strain gage fixed to the contact lens, the active strain gage being placed at a distance from the center of the contact lens and being not in direct contact with the eye, wherein the active strain gage comprises a polygonal portion situated around the center of the contact lens.

The manufacturing of the strain gage of the invention is greatly facilitated, because the polygonal portion is made of a suite of rectilinear elements that are relatively easy to manufacture in a regular and reproducible manner. The strain gage of the invention being made essentially of rectilinear segments, its performance and manufacturing repeatability are high, while the scrap factor can be kept very low with all suitable manufacturing processes. Furthermore, by adequately choosing the shape of the polygonal portion, the proportion of waste material can be greatly reduced when several gages are manufactured simultaneously on a single substrate.

The polygonal portion of the gage situated around the center of the lens approximately follows a virtual circle on the surface of the eyeball when the lens is correctly put in place. The polygonal gage, through the deformation of the soft contact lens on which it is fixed, is thus subjected to the strain due to the peripheral deformations of the eyeball, which are correlated with intraocular pressure (IOP).

The present invention will be better understood with the help of the following description illustrated by the figures, whereas:
Figure 1 shows an intraocular pressure monitoring device according to a preferred embodiment of the invention.
Figure 2 shows an intraocular pressure monitoring device according to another preferred embodiment of the invention.
Figure 3 illustrates the manufacturing of several strain gages of the invention on a single substrate.
Figure 4 illustrates another pattern for manufacturing several polygonal strain gages of the invention on a single substrate.
Figure 5 illustrates a pattern for manufacturing strain gages according to another embodiment of the invention.
Figure 6A shows a folded strain gage according to another embodiment of the invention.
Figure 6B shows the strain gage of figure 6A once unfolded.

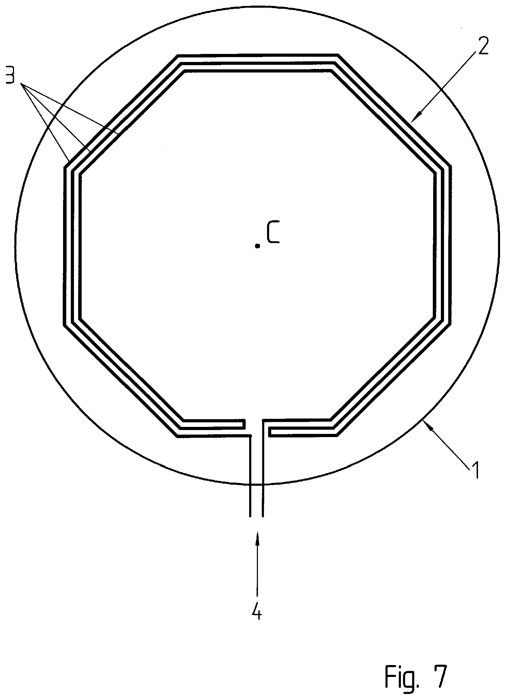

Figure 7 shows an intraocular pressure monitoring device according to another embodiment of the invention.
Figure 8 shows a simplified block diagram of an intraocular pressure monitoring system comprising an intraocular pressure monitoring device according to the invention with an embedded telemetry system and extracorporal receiving units.

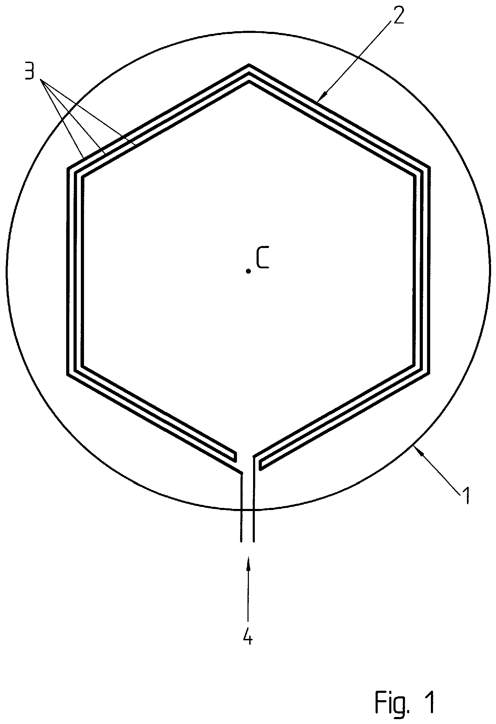

In a preferred embodiment illustrated in Fig. 1, the intraocular pressure monitoring device of the invention comprises a contact lens 1, preferably a soft contact lens, with an active strain gage 2 disposed around the lens center C.

The active strain gage 2 is preferably made of a continuous longitudinal element, or wire, made at least partly of a resistive metal, the gage resistance varying according to the gage strain. Both ends 4 of the wire are connected to a data transmission system (not illustrated). The transmission is for example achieved via a wireless telemetry system.

According to the invention, a portion 3 of the active strain gage 2 is polygonal, i.e. it comprises longitudinally aligned rectilinear segments oriented approximately tangentially to the lens center C, thus forming at least a part of a polygon.

For a variable resistance pressure gage, in order to have a more accurate measurement, the gage resistance is maximized and its grid area preferably covers all the zones that have to be monitored. In the present invention, this is achieved for example by folding the continuous longitudinal element, or wire, into several portions which are arranged parallel to each other. In the illustrated embodiment, the longitudinal element forming the active strain gage 2 is folded such that several of its rectilinear segments are parallel to each other, thus forming concentric polygonal portions 3.

In a preferred embodiment, the active strain gage is a Micro-Electro-Mechanical System (MEMS), for example a foil strain gage comprising a substrate on which a metallic layer is deposited or laminated and patterned by wet or dry etch in a desired configuration. The substrate is made for example of a polymer (e.g. polymide) or epoxy resin, while the metallic layer is of any resistive or semiconductor material. Preferably, the substrate is polyimide, while the metallic layer is platinum. Polyimide as substrate is particularly suitable because it is widely used in MEMS technology and it is biocompatible, as well as platinum which also has a good strain gage factor.

MEMS strain gages are manufactured according to Integrated Circuit manufacturing processes. An advantage of this manufacturing process is that every parameter of the strain gage, in particular the thickness of the metallic strain gage layer, can be controlled very precisely. The design of the grid can be realized with a precision of about 1µm and gives the possibility to build really specific gages. Moreover the process is completely and easily reproducible.

Other manufacturing processes are however possible for building the strain gage of the device of the invention. The strain gage can for example be manufactured by embossment and/or cutting of a resistive foil or of a substrate with a resistive overlay, by bending and forming of a thin wire of a resistive material, for example of a metallic wire having a diameter between 0.01 mm and 0.1 mm, etc. In all cases, the fact that the strain gage of the invention mainly comprises rectilinear elements makes it relatively easy and thus cheap to manufacture.

According to the invention, the precision of the design and the reproducibility, thus the quality of the strain gage, is improved due to the fact that the strain gage essentially comprises rectilinear elements that are relatively easy to manufacture. The strain gage of the device of the invention being easy to manufacture, the scrap rate can also be kept very low with all suitable manufacturing processes.

The gage can be fixed to the lens by any method. It can be first fixed to a substrate which is then fixed on or embedded in the lens or it can be directly fixed to or embedded in the lens.

The active gage can be placed at any distance from the center of the contact lens. In a preferred embodiment, the active gage is shaped in order to be placed on the corneoscleral junction which is a zone where changes in IOP induce maximum corneal deformation.

According to the invention, the intraocular pressure monitoring device can comprise two or more active gages on the contact lens. The polygonal sections of the several strain gages can be placed in different sectors of the same circumference of the lens, or they can form several concentric polygons or parts of polygon.

The intraocular pressure monitoring device of the invention furthermore advantageously comprises passive gages for thermal compensation. The passive gages are preferably made of a continuous longitudinal element comprising several rectilinear sections radially arranged next to each other on the contact lens. The passive gages are thus not subjected to the spherical deformations of the eyeball, but only to the dilatation and contraction of the lens due to the temperature changes. The passive gages thus allow accurate measurement of the variations of the strain induced by the temperature variations only.

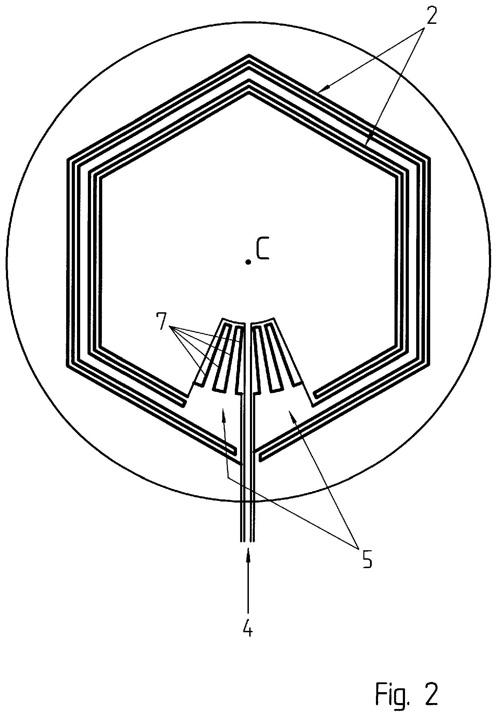

In a preferred embodiment illustrated in Fig. 2, the intraocular pressure monitoring device comprises four gages in a Wheatstone Bridge configuration, wherein two active gages and two passives gages are placed alternatively on the bridge.

The passive gages 5 are made of a continuous longitudinal element, or wire, folded into several rectilinear portions 7 that are radially arranged relative to the lens 1, i.e. their longitudinal axis cross the lens center C. The wire portions of active and passive gages can be very close to each other in order to minimize the gage area, or more spaced in order to maximize thermal exchanges and gage area.

In this configuration, the two active strain gages 2 measure one type of strain (the strongest one) and double the sensitivity of the measure on the Wheatstone Bridge. The two passive gages 5 compensate for thermal derivation if active and passive gages have the same resistance value when no stress is applied.

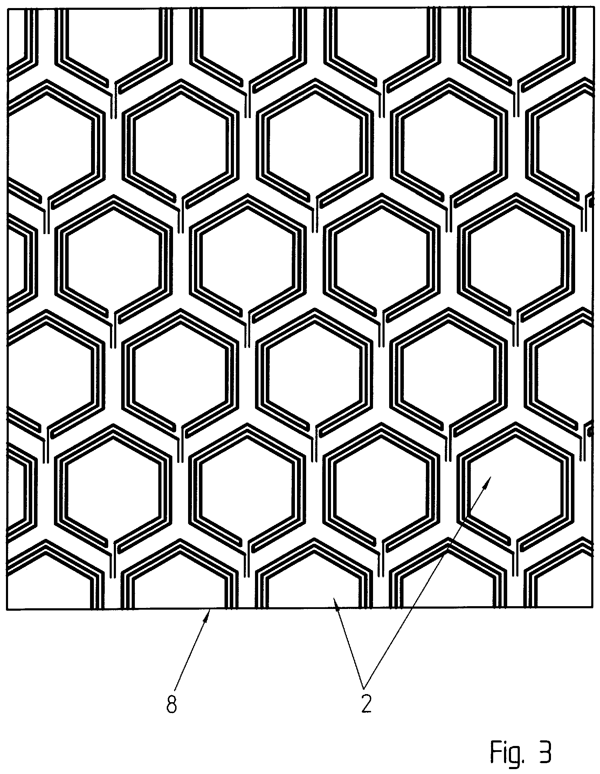

As illustrated in Fig. 3, several strain gages 2 of the invention, for example MEMS strain gages, can be manufactured simultaneously on a single substrate 8. Thanks to the generally polygonal shape of the gages 2, the free space between neighboring gages 2 can be minimized, thus reducing the proportion of waste material and the production cost of each gage.

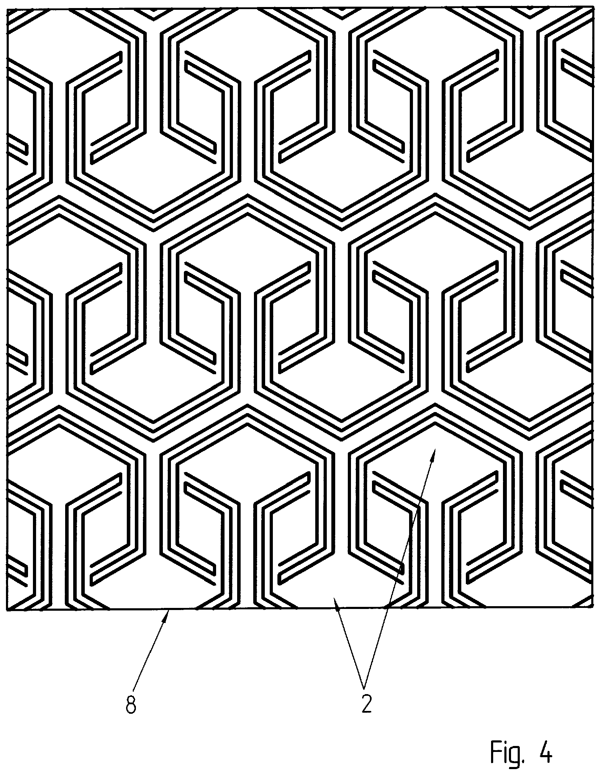

Fig. 4 shows another arrangement for manufacturing several gages 2 on a single substrate 8, where the proportion of waste material is minimized by imbricating the polygonal gages 2 into each other, the end portion of two different gages being formed on the surface located within a third polygonal gage.

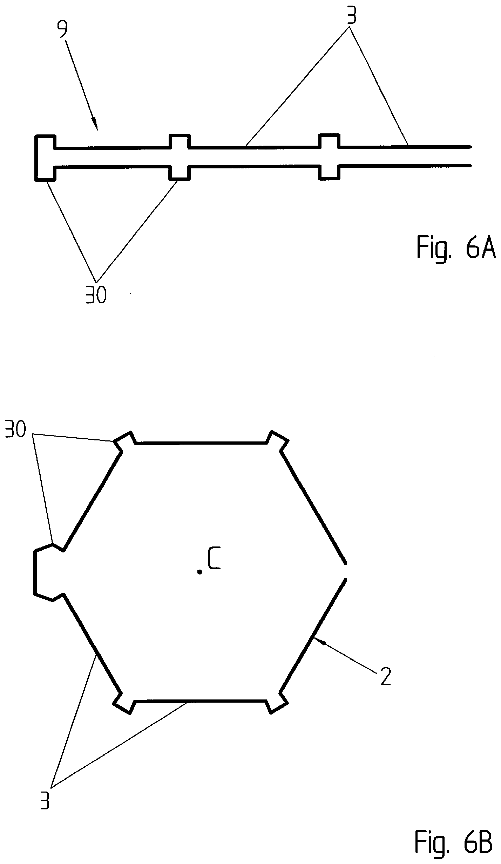

Fig. 6A and 6B illustrate another embodiment of the invention, where the polygonal gage 2 is made by bending a preformed wire 9. In the illustrated example, the preformed wire 9 comprises six rectilinear segments 3 arranged in two lines of three segments each. The rectilinear segments 3 are separated from each other by flexible zones 30 allowing the unfolding of the preformed wire 9 into a hexagon, without bending the rectilinear segments 3. The flexible zones 30 are for example formed by relatively short segments arranged to form a part of a rectangle.

The preformed wire 9 is formed for example by embossment, etching or any other appropriate manufacturing method. The preformed wire being made only of rectilinear segments disposed at right angles to each other, its reliable manufacturing is relatively easy. The preformed wire 9 is then unfolded by bending it into its flexible zone in order to obtain the desired polygonal gage 2 of the invention, as illustrated in Fig. 6B.

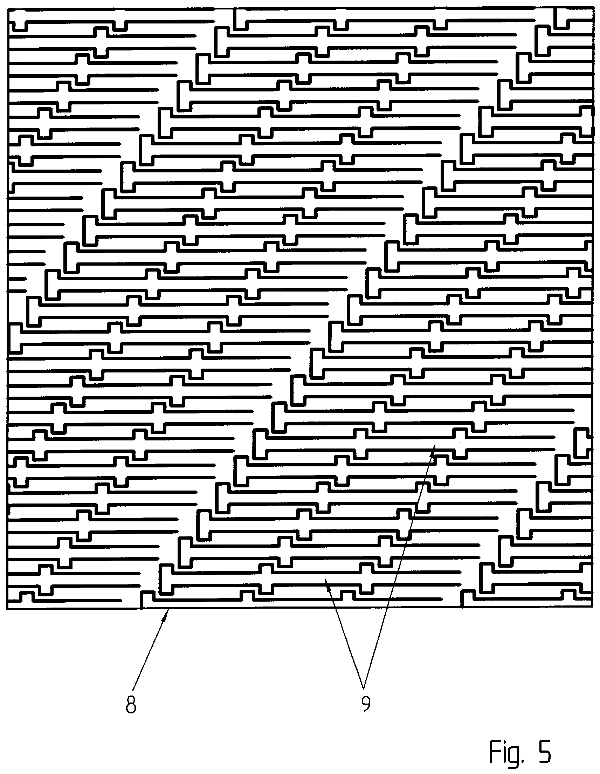

Fig. 5 illustrates the manufacturing of a plurality of preformed wire 9 on a single substrate 8. Thanks to the longitudinal placement of the rectilinear segments 3, the preformed wires 9 have a generally longitudinal shape allowing them to be arranged very close to each other on the substrate 8, reducing even more the proportion of waste material.

In the embodiments described above and illustrated in Fig. 1-6B, the polygonal section of the strain gage forms a part of a hexagon. Other polygonal shapes are possible within the frame of the invention. The polygonal section of the strain gage can for example form a part of an octagon, as illustrated by way of example in Fig. 4, a decagon, a dodecagon, etc. The smaller the rectilinear segments are, the closer the polygonal section is to a circle, while still consisting of rectilinear segments, thus benefiting of the advantages mentioned above.

The data transmission from the gage can be achieved by using a wire transmission or, preferably, a wireless transmission system.

In addition to the gage the contact lens can further comprise other measuring devices such as an ElectroRetinoGraph or a chemical analysis sensor.

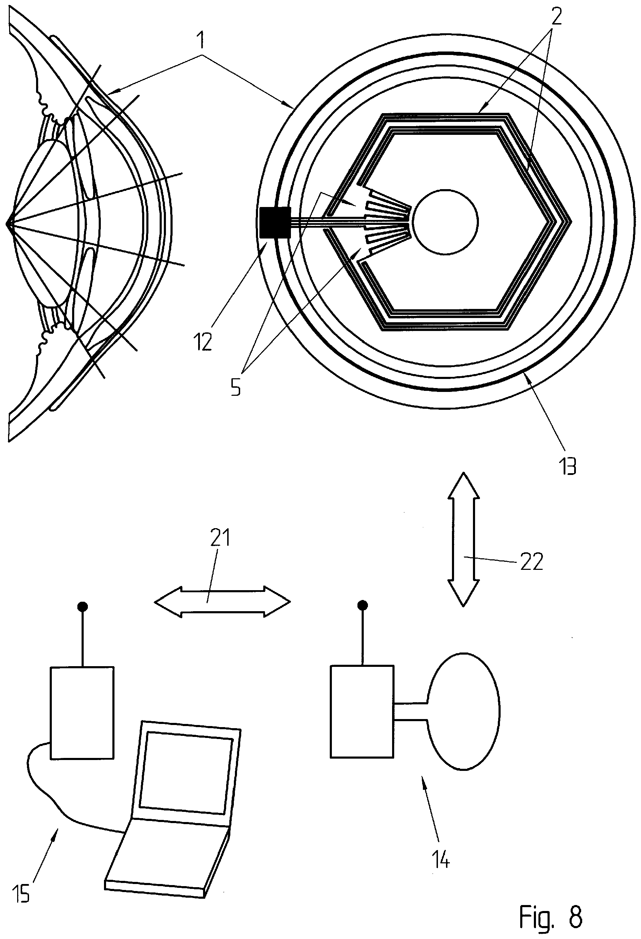

Fig. 8 shows the simplified block diagram of a preferred configuration of an entire intraocular pressure monitoring system with an embedded telemetry system and extracorporal receiving units. The contact lens 1 comprises a pressure sensor, i.e. active gages 2 and passive gages 5 in a Wheatstone Bridge configuration, a low-power transponder 12 and a loop antenna 13.

Powering and communication are performed contactlessly between the transponder and an extracorporal mobile interrogation unit (MIU) 14 via coupled loop antennas. The MIU 14 provides the sensor with energy via the thus formed first RF link 22 and passes the received transponder data to a stationary data receiver (SDR) 15, for example via a second RF link 21. The SDR 15 completes the monitoring setup. It stores and displays the received data.

## Claims

1. Intraocular pressure monitoring device comprising a soft contact lens (1) such as a silicone contact lens and an active strain gage (2) fixed to said contact lens (1), said active strain gage (2) being placed at a distance from the center (C) of the contact lens and being not in direct contact with the eye, **characterized in that** said active strain gage comprises a polygonal portion situated around said center (C) of the contact lens.

2. Intraocular pressure monitoring device according to claim 1 wherein said active strain gage (2) is made of a resistive material, such as a metal or an alloy.

3. Intraocular pressure monitoring device according to any of claims 1 or 2 wherein said active strain gage (2) is a continuous element.

4. Intraocular pressure monitoring device according to claim 3 wherein said continuous element is placed in such a way that several polygonal portions (3) are disposed parallel to each other.

5. Intraocular pressure monitoring device according to any of claims 1 to 4 further comprising a passive strain gage (5) placed on said contact lens (1).

6. Intraocular pressure monitoring device according to claim 5 wherein said passive strain gage (5) comprises several rectilinear portions (7) radially arranged on said contact lens (1).

7. Intraocular pressure monitoring device according to any of claims 1 to 6 wherein said active strain gage (2) is shaped in order to be placed on the corneosclera junction.

8. Intraocular pressure monitoring device according to any of claims 1 to 7 including a wireless telemetry system (12-15) for data transmission with said strain gage (2, 5).

9. Intraocular pressure monitoring device according to any of claims 1 to 8 wherein said active strain gage (2) is microfabricated.

10. Intraocular pressure monitoring device according to any of claims 1 to 8 wherein said active strain gage (2) is a wire.

11. Intraocular pressure monitoring device according to any of claims 1 to 10 wherein said contact lens (1) further comprises other measuring devices such as an ElectroRetinoGraph or a chemical analysis sensor.

12. Intraocular pressure monitoring device according to any of claims 1 to 11 wherein it comprises several active gages (2).

13. Intraocular pressure monitoring device according to any of claims 1 to 12 wherein it comprises several passive gages (5).

14. Intraocular pressure monitoring device according to any of claims 1 to 13 wherein it comprise four gages in a Wheatstone bridge configuration, such as two active gages (2) and two passives gages (5) being placed alternatively on the bridge.

## Patentansprüche

1. Vorrichtung zur Überwachung des Augeninnendrucks mit einer weichen Kontaktlinse (1) wie einer Silikonkontaktlinse und einem an der besagten Kontaktlinse (1) befestigten aktiven Dehnungsmessstreifen (2), wobei der besagte aktive Dehnungsmessstreifen (2) in einem Abstand von der Mitte (C) der Kontaktlinse angeordnet ist und nicht in direktem Kontakt mit dem Auge steht, **dadurch gekennzeichnet, dass** der besagte aktive Dehnungsmessstreifen einen um den besagten Mittelpunkt (C) der Kontaktlinse herum angeordneten polygonalen Abschnitt aufweist.

2. Vorrichtung zur Überwachung des Augeninnendrucks gemäss Anspruch 1, wobei der besagte aktive Dehnungsmessstreifen (2) aus einem Widerstandsmaterial wie einem Metall oder einer Legierung hergestellt ist.

3. Vorrichtung zur Überwachung des Augeninnendrucks gemäss irgendeinem der Ansprüche 1 oder 2, wobei der besagte aktive Dehnungsmessstreifen (2) ein kontinuierliches Element ist.

4. Vorrichtung zur Überwachung des Augeninnendrucks gemäss Anspruch 3, wobei das besagte kontinuierliche Element so angeordnet ist, dass mehrere polygonale Abschnitte (3) parallel zueinander angeordnet sind.

5. Vorrichtung zur Überwachung des Augeninnendrucks gemäss irgendeinem der Ansprüche 1 bis 4, ferner mit einem passiven Dehnungsmessstreifen (5), der auf der besagten Kontaktlinse (1) angeordnet ist.

6. Vorrichtung zur Überwachung des Augeninnendrucks gemäss Anspruch 5, wobei der besagte passive Dehnungsmessstreifen (5) mehrere geradlinige Abschnitte (7) aufweist, die radial auf der besagten Kontaktlinse (1) angeordnet sind.

7. Vorrichtung zur Überwachung des Augeninnendrucks gemäss irgendeinem der Ansprüche 1 bis 6, wobei der besagte aktive Dehnungsmessstreifen (2) so geformt ist, dass er auf den Hornhautanschluss aufgesetzt wird.

8. Vorrichtung zur Überwachung des Augeninnendrucks gemäss irgendeinem der Ansprüche 1 bis 7, mit einem drahtlosen Telemetriesystem (12-15) zur Datenübertragung mit dem besagten Dehnungsmessstreifen (2, 5).

9. Vorrichtung zur Überwachung des Augeninnendrucks gemäss irgendeinem der Ansprüche 1 bis 8, wobei der besagte aktive Dehnungsmessstreifen (2) mikrogefertigt ist.

10. Vorrichtung zur Überwachung des Augeninnendrucks gemäss irgendeinem der Ansprüche 1 bis 8, wobei der besagte aktive Dehnungsmessstreifen (2) ein Draht ist.

11. Vorrichtung zur Überwachung des Augeninnendrucks gemäss irgendeinem der Ansprüche 1 bis 10, wobei die besagte Kontaktlinse (1) ferner andere Messvorrichtungen wie einen ElectroRetinoGraph oder einen chemischen Analysensensor umfasst.

12. Vorrichtung zur Überwachung des Augeninnendrucks gemäss irgendeinem der Ansprüche 1 bis 11, wobei sie mehrere aktive Messstreifen (2) aufweist.

13. Vorrichtung zur Überwachung des Augeninnendrucks gemäss irgendeinem der Ansprüche 1 bis 12, wobei sie mehrere passive Messstreifen (5) aufweist.

14. Vorrichtung zur Überwachung des Augeninnendrucks gemäss irgendeinem der Ansprüche 1 bis 13, wobei sie vier Messstreifen in einer Wheatstone-Brückenkonfiguration umfasst, wie z. B. zwei aktive Messstreifen (2) und zwei passive Messstreifen (5), die abwechselnd auf der Brücke angeordnet sind.

## Revendications

1. Dispositif de surveillance de la pression intraoculaire comprenant une lentille de contact souple (1) telle qu'une lentille de contact en silicone et une jauge de contrainte active (2) fixée à ladite lentille de contact (1), ladite jauge de contrainte active (2) étant placée à distance du centre (C) de la lentille de contact et n'étant pas en contact direct avec l'oeil, **caractérisé en ce que** ladite jauge de contrainte active comprend une portion polygonale située autour dudit centre (C) de la lentille de contact.

2. Dispositif de surveillance de la pression intraoculaire selon la revendication 1, dans lequel ladite jauge de contrainte active (2) est constituée d'un matériau résistif, tel qu'un métal ou un alliage.

3. Dispositif de surveillance de pression intraoculaire selon l'une quelconque des revendications 1 ou 2, dans lequel ladite jauge de contrainte active (2) est un élément continu.

4. Dispositif de surveillance de la pression intraoculaire selon la revendication 3, dans lequel ledit élément continu est placé de telle sorte que plusieurs portions polygonales (3) sont disposées parallèlement l'une à l'autre.

5. Dispositif de surveillance de la pression intraoculaire selon l'une quelconque des revendications 1 à 4, comprenant en outre une jauge de contrainte passive (5) placée sur ladite lentille de contact (1).

6. Dispositif de surveillance de la pression intraoculaire selon la revendication 5, dans lequel ladite jauge de contrainte passive (5) comprend plusieurs portions rectilignes (7) disposées radialement sur ladite lentille de contact (1).

7. Dispositif de surveillance de la pression intraoculaire selon l'une quelconque des revendications 1 à 6, dans lequel ladite jauge de contrainte active (2) est configurée pour être placée sur la jonction cornéosclérale.

8. Dispositif de surveillance de la pression intraoculaire selon l'une quelconque des revendications 1 à 7, comprenant un système de télémétrie sans fil (12-15) pour la transmission de données avec ladite jauge de contrainte (2, 5).

9. Dispositif de surveillance de la pression intraoculaire selon l'une quelconque des revendications 1 à 8, dans lequel ladite jauge de contrainte active (2) est micro-fabriquée.

10. Dispositif de surveillance de la pression intraoculaire selon l'une quelconque des revendications 1 à 8, dans lequel ladite jauge de contrainte active (2) est un fil.

11. Dispositif de surveillance de pression intraoculaire selon l'une quelconque des revendications 1 à 10, dans lequel ladite lentille de contact (1) comprend en outre d'autres dispositifs de mesure tels qu'un ElectroRetinoGraph ou un capteur d'analyse chimique.

12. Dispositif de surveillance de la pression intraoculaire selon l'une quelconque des revendications 1 à 11, comprenant plusieurs jauges actives (2).

13. Dispositif de surveillance de la pression intraoculaire selon l'une quelconque des revendications 1 à 12, comprenant plusieurs jauges passives (5).

14. Dispositif de surveillance de la pression intraoculaire selon l'une quelconque des revendications 1 à 13, comprenant quatre jauges dans une configuration de pont de Wheatstone, tels que deux jauges actives (2) et deux jauges passives (5) étant placées alternativement sur le pont.
